# EUROPEAN PATENT SPECIFICATION

(11) **EP 4 763 110 B1**
(45) Date of publication and mention of the grant of the patent: **30.09.2026**
(21) Application number: 24223006.8
(22) Date of filing: 23.12.2024
(51) Int. Cl.: A61B 17/70

(54) **BONE ANCHORING DEVICE FOR COUPLING A ROD TO A BONE ANCHORING ELEMENT**
KNOCHENVERANKERUNGSVORRICHTUNG ZUR KOPPLUNG EINER STANGE AN EIN KNOCHENVERANKERUNGSELEMENT
DISPOSITIF D'ANCRAGE OSSEUX POUR COUPLER UNE TIGE À UN ÉLÉMENT D'ANCRAGE OSSEUX

(43) Date of publication of application: 24.06.2026
(73) Proprietor: Biedermann Technologies GmbH & Co. KG, 78166 Donaueschingen (DE)
(72) Inventor: BIEDERMANN, Lutz, 78048 VS-Villingen (DE); BIEDERMANN, Timo, 78647 Trossingen (DE); FISCHER, Bernd, 79877 Friedenweiler (DE); DANNECKER, Berthold, 78112 St. Georgen (DE)
(74) Representative: Prüfer & Partner mbB Patentanwälte · Rechtsanwälte

(56) References cited:
- EP-A1- 4 437 983
- US-A1- 2010 305 621
- US-A1- 2018 008 319

## Description

The invention relates to a bone anchoring device comprising a bone anchoring element and a coupling device for coupling a rod to the bone anchoring element. In particular, the bone anchoring device is a modular bone anchoring device that can be selectively adapted for various clinical requirements.

Bone anchoring devices comprising a bone anchoring element and a receiving part are used in orthopaedic surgery, in particular in spinal surgery, for coupling a rod to the bone anchoring element that is anchored in bone or a vertebra and for connecting several bone anchoring devices by the rod. In a polyaxial bone anchoring device, a head of the bone anchoring element is pivotably received in the receiving part, so that the receiving part can assume various angular positions in multiple different planes with respect to the bone anchoring element. Thus, a suitable orientation of the bone anchoring element with respect to the rod can be achieved.

Depending on the use or clinical application, it can be desirable to provide a bone anchoring device configured to restrict pivoting of the bone anchoring element with respect to the receiving part to a single plane, i.e. a uniplanar bone anchoring device, or to provide a monoaxial bone anchoring device in which the shank has a fixed angle, preferably a zero-angle, with respect to the receiving part.

Various designs of uniplanar bone anchoring devices are known in which the shank of the bone anchoring element can pivot in the receiving part only in a single plane. For example, US 7,749,258 B2 describes a uniplanar bone anchoring device including a receiving part for receiving a rod, a pressure element and a bone anchoring element being moveable relative to the receiving part in a limited angular range about the longitudinal axis of the receiving part, the angles lying in a single plane. The movement of the bone anchoring element relative to the receiving part is limited by a form-fit connection including cooperating guiding surfaces at the head of the bone anchoring element and at the pressure element.

US 7,892,259 B2 describes a bone anchoring device including a receiving part having a bore and an anchoring element having a head positionable within the bore, the head having a spherically shaped surface portion and recessed surface portions. Pins are insertable into through-holes formed in the receiving part to engage the recessed surface portions of the head such that the anchoring element is pivotable relative to the receiving part around a single axis of rotation.

US 2018/008319 A1 describes a bone anchor assembly comprising a bone anchor with a head having two opposed flat side surfaces, a receiving member for receiving a spinal rod and the head of the bone anchor, and a restriction member configured to mate with the flat side surfaces of the head to prevent pivoting of the shaft about an axis that is parallel to the flat side surfaces.

There is still a need to provide a bone anchoring device having a simple structure with only few parts that may facilitate implementation of at least a polyaxial and/or a uniplanar and/or a monoaxial bone anchoring device. Thus, it is the object underlying the invention to provide a bone anchoring device that can be used in a modular manner enabling a selectable functionality in terms of a polyaxial, a monoaxial and/or a uniplanar design.

The object is solved by a bone anchoring device according to claim 1. Further developments are given in the dependent claims.

According to an embodiment of the invention a bone anchoring device is provided that comprises a bone anchoring element having a shank for anchoring to bone and a head, and a coupling device for coupling a rod to the bone anchoring element. The coupling device comprises a receiving part having a first end, an opposite second end, and a passage extending between the first end and the second end, the passage defining a central axis, wherein the receiving part comprises an accommodation space at the second end for accommodating the head of the bone anchoring element. The coupling device further comprises a pressure element configured to be arranged in the passage of the receiving part and to exert pressure onto the head received within the accommodation space to clamp the head at a particular angular position relative to the receiving part, wherein the pressure element comprises at least one opening that provides access to the inserted head from a direction transverse to the central axis, and at least one restraining element or also designated restriction element configured to be arranged in the passage of the receiving part, wherein the restraining element is movable relative to the pressure element in an axial direction between a first position and a second position, wherein in the second position the restraining element contacts the inserted head through the opening of the pressure element to restrict pivoting of the inserted head relative to the receiving part.

Transverse to the central axis means that the direction in which the inserted head is accessible intersects the central axis, preferably the direction is substantially perpendicular, further preferably perpendicular, to the central axis.

Preferably, when the restraining element is in the first position, it may be free from any interaction with the inserted head, i.e., it does not act upon an inserted head, and/or when the restraining element is in the second position, it may may directly contact the inserted head to restrict pivoting of the head relative to the receiving part. For example, when the restraining element is in the first position, the resulting bone anchoring device may be a polyaxial bone anchoring device, and when the restraining element is in the second position, the resulting bone anchoring device may be a uniplanar bone anchoring device. More generally, the restraining element may have a first restraining surface and the head may have a second restraining surface cooperating with the first restraining surface to reduce a degree of freedom of pivoting of the head with respect to the receiving part.

With the bone anchoring device, it is possible to selectively use the bone anchoring device as a polyaxial bone anchoring device or as a uniplanar bone anchoring device. The uniplanar bone anchoring device can be implemented by applying at least one restraining element to restrict the pivoting motion of the head relative to the receiving part to a single plane. This can be carried out at any time before surgery. Thus, the bone anchoring device requires only few parts and the inventory required for surgery can be reduced, while providing a variety of different possible applications.

Since the receiving part may be more expensive and/or more difficult to manufacture compared to the bone anchoring element, the manufacturing costs and/or the costs for stock holding can be decreased if the same coupling device can be used for a polyaxial or a uniplanar application.

Preferably, the restraining element is only accessible within the passage from above, i.e. from a direction of the first end of the receiving part. An axial displacement of the restraining element may be effected by an instrument that enters the passage of the receiving part to act upon the restraining element. In particular, the restraining element may not be accessible or actuatable laterally from the outside of the receiving part, i.e. in a direction transverse to the central axis.

Preferably, the receiving part and/or the pressure element and/or the restraining element each is a monolithic piece.

According to a further embodiment, a bone anchoring device is provided that comprises a bone anchoring element having a shank for anchoring to bone and a head, and a coupling device for coupling a rod to the bone anchoring element. The coupling device comprises a receiving part having a first end, an opposite second end, and a passage extending between the first end and the second end, the passage defining a central axis, wherein the receiving part comprises an accommodation space at the second end for accommodating the head of the bone anchoring element. The coupling device further comprises a pressure element configured to be arranged in the passage of the receiving part and to exert pressure onto the head received within the accommodation space to clamp the head at a particular angular position relative to the receiving part, and a plug member that is configured to cooperate with the inserted head of the bone anchoring element such that the plug member forms a monoaxial connection in which the bone anchoring element is prevented from pivoting relative to the receiving part.

This makes it possible to use a bone anchoring device that has been originally designed as a polyaxial or uniplanar bone anchoring device for various monoaxial applications, such as trauma applications, growing rod applications, posterior scoliosis correction, anterior scoliosis surgery or anterior scoliosis tethering.

Further features and advantages will become apparent from the description of embodiments by means of the accompanying drawings. In the drawings:
- Fig. 1: shows a perspective exploded view of a bone anchoring device according to a first embodiment.
- Fig. 2: shows a perspective view of the bone anchoring device of Fig. 1 in an assembled state.
- Fig. 3: shows a cross-sectional view of the bone anchoring device of Figs. 1 and 2, the cross-section taken in a plane extending through the central axis and perpendicular to the rod axis of an inserted rod.
- Fig. 4: shows a perspective view from a top of a receiving part of the bone anchoring device of Figs. 1 to 3.
- Fig. 5: shows a perspective view from a bottom of the receiving part of Fig. 4.
- Fig. 6: shows a top view of the receiving part of Figs. 4 and 5.
- Fig. 7: shows a cross-sectional view of the receiving part of Figs. 4 to 6, the cross-section taken along line A-A in Fig. 6.
- Fig. 8: shows a perspective view from a top of a pressure element of the bone anchoring device of Figs 1 to 3.
- Fig. 9: shows a perspective view from a bottom of the pressure element of Fig. 8.
- Fig. 10: shows a top view of the pressure element of Figs. 8 and 9.
- Fig. 11: shows a cross-sectional view of the pressure element of Figs. 8 to 10, the cross-section taken along line B-B in Fig. 10.
- Fig. 12: shows a perspective view from a top of a restraining element of the bone anchoring device of Figs. 1 to 3.
- Fig. 13: shows a further perspective view from the top of the restraining element of Fig. 12.
- Fig. 14: shows a side view of the restraining element of Figs. 12 and 13.
- Fig. 15: shows a perspective view from a top of a plug member of the bone anchoring device of Figs. 1 to 3.
- Fig. 16: shows a side view of the plug member of Fig. 15.
- Fig. 17: shows a perspective view from a bottom of the plug member of Figs. 15 and 16.
- Fig. 18a: shows a cross-sectional view of the bone anchoring device of Figs. 1 to 3 without the plug member and with the pressure element in an insertion position before inserting the head into the receiving part.
- Fig. 18b: shows a cross-sectional view of the bone anchoring device of Fig. 18a with the head inserted into the receiving part.
- Fig. 18c: shows a cross-sectional view of the bone anchoring device of Fig. 18b with the pressure element in a pre-locking position and the restraining element in an upper position.
- Fig. 18d: shows a cross-sectional view of the bone anchoring device of Figs. 18b and 18c with the pressure element in the pre-locking position and the restraining element in a lower position.
- Fig. 19: shows a perspective exploded view of a bone anchoring device according to a second embodiment.
- Fig. 20: shows a cross-sectional view of the bone anchoring device of Fig. 19, the cross-section taken in a plane extending through the central axis and perpendicular to the rod axis of an inserted rod.
- Fig. 21: shows a perspective view from a top of a receiving part of the bone anchoring device of Figs. 19 and 20.
- Fig. 22: shows a perspective view from a bottom of the receiving part of Fig. 21.
- Fig. 23: shows a top view of the receiving part of Figs. 21 and 22.
- Fig. 24: shows a cross-sectional view of the receiving part of Figs. 21 to 23, the cross-section taken along line D-D in Fig. 23.
- Fig. 25: shows a perspective view from a top of a pressure element of the bone anchoring device of 19 and 20.
- Fig. 26: shows a perspective view from a bottom of the pressure element of Fig. 25.
- Fig. 27: shows a top view of the pressure element of Figs. 25 and 26.
- Fig. 28: shows a cross-sectional view of the pressure element of Figs. 25 to 27, the cross-section taken along line E-E in Fig. 27.
- Fig. 29: shows a perspective view from a top of a restraining element of the bone anchoring device of Figs. 19 and 20.
- Fig. 30: shows a further perspective view from the top of the restraining element of Fig. 29.
- Fig. 31: shows a side view of the restraining element of Figs. 29 and 30.
- Fig. 32a: shows a cross-sectional view of the bone anchoring device of Figs. 19 and 20 with the pressure element in an insertion position prior to inserting the head into the receiving part.
- Fig. 32b: shows a cross-sectional view of the bone anchoring device of Fig. 32a with the head inserted into the receiving part.
- Fig. 32c: shows a cross-sectional view of the bone anchoring device of Fig. 32b with the restraining element in an upper position.
- Fig. 32d: shows a cross-sectional view of the bone anchoring device of Figs. 32b and 32c with the restraining element in a lower position.

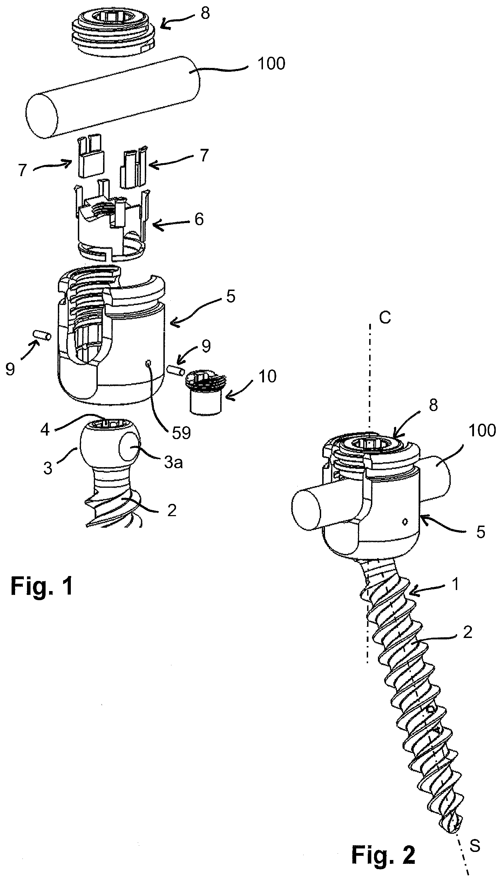

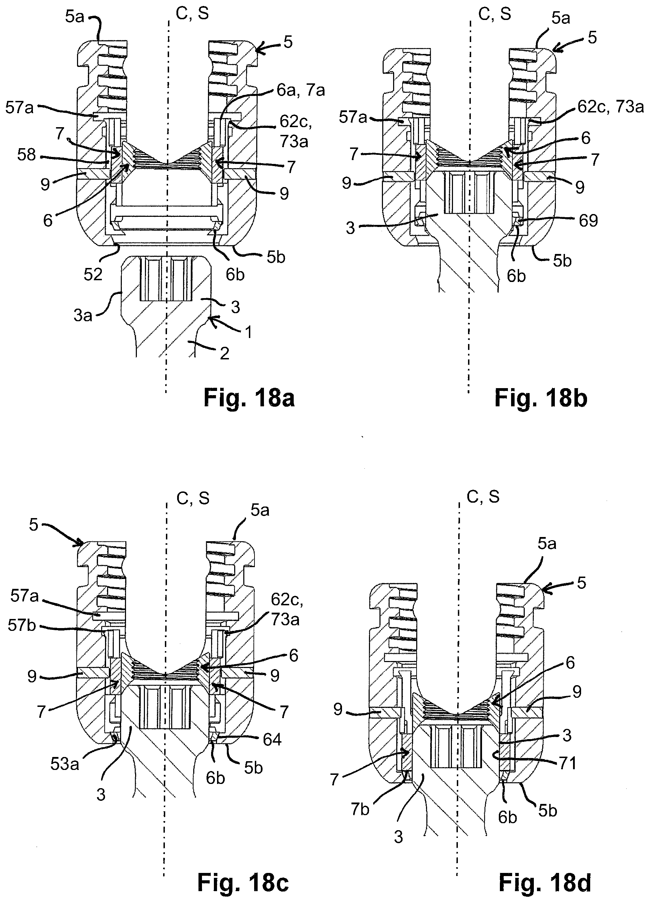

With reference to Figs. 1 to 18d, a first embodiment of a bone anchoring device is described. As shown in particular in Figs. 1 to 3, the bone anchoring device includes a bone anchoring element 1 having a shank 2 for anchoring in bone or in a vertebra and a head 3. The shank 2 defines a shank axis S and has a bone engagement feature, such as a thread. Further, the bone anchoring device includes a receiving part 5 for receiving the head 3 of the bone anchoring element 1 and for receiving a rod 100 that is configured to connect several bone anchoring devices. A pressure element 6 is configured to be arranged within the receiving part 5 for exerting pressure onto the head 3 of the bone anchoring element 1 and for clamping the head 3 with the shank 2 having a particular angular position relative to the receiving part 5.

Two restraining elements 7 are configured to be arranged within the receiving part 5 so as to be movable relative to the pressure element 6 between a first position and a second position in the axial direction. In the first position, the restraining elements do not act upon the inserted head 3. In particular, when the restraining elements 7 are in the first position, the shank 2 of the bone anchoring element 1 may be pivotable in multiple different planes relative to the receiving part 5, i.e., the resulting bone anchoring device is a polyaxial bone anchoring device. When the restraining elements 7 are in the second position, the restraining elements may directly contact the inserted head 3 to restrict pivoting of the shank 2 relative to the receiving part 5 within a single plane, i.e., the resulting bone anchoring device is a uniplanar bone anchoring device. The single pivot plane may be defined by the cooperation of a first restraining surface provided at the restraining elements 7 and a second restraining surface provided at the head 3, as described further below. Optionally, a plug member 10 is provided that is configured to be inserted into the pressure element 6 and to cooperate with the head 3 such that the bone anchoring element 1 is prevented from pivoting relative to the receiving part 5, i.e., the resulting bone anchoring device is a monoaxial bone anchoring device.

Further, in order to secure the pressure element 6 against rotation, two pins 9 are provided that are insertable into transverse through-holes 59 provided at the receiving part 5. To lock the head 3 and the rod 100 in the receiving part 5, a locking element 8 in the form of an inner screw or set screw is provided.

The head 3 of the bone anchoring element 1 comprises a spherically-shaped outer surface portion and two second restraining surfaces in the form of substantially planar or flat surface portions 3a provided at the outer surface portion that are configured to cooperate with the first restraining surfaces of the restraining elements 7. The planar surface portions 3a provide for a reduced diameter of the head 3 as compared to a diameter defined by the spherically-shaped outer surface portion, i.e., they are recessed from an ideal sphere as defined by the spherically-shaped outer surface portion. The planar surface portions 3a are located at opposite sides of the head and are offset at 180° from one another. A plane of the planar surface portions 3a may be substantially parallel to the shank axis S and the two planar surface portions 3a may be substantially parallel to one another.

The head 3 further comprises at its free end opposite to the shank 2 an engagement recess 4 for engagement with a tool, such as a driver.

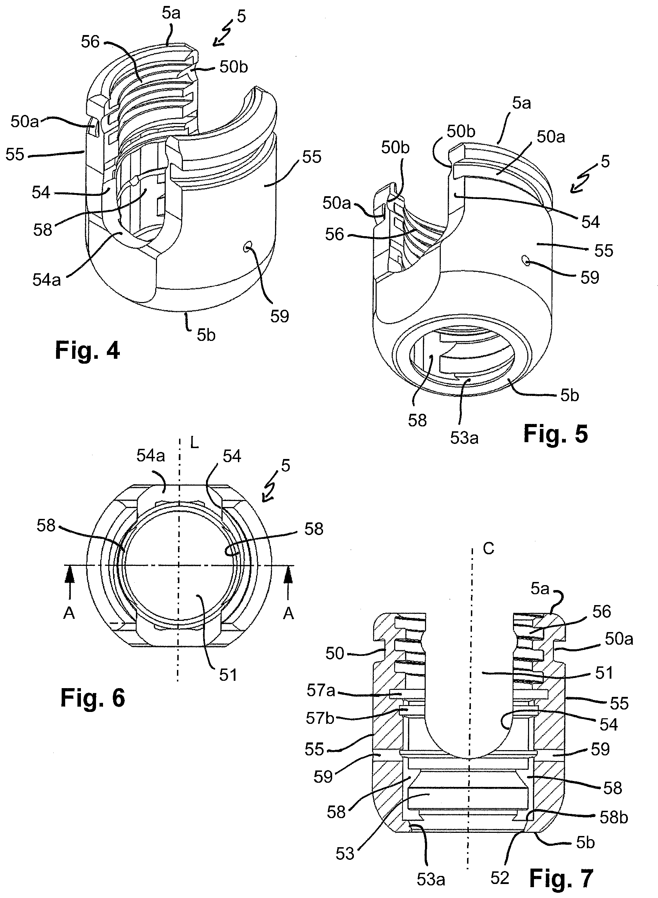

Referring in addition to Figs. 4 to 7, the receiving part 5 is described. The receiving part 5, which may be a monolithic piece, has a first end or top end 5a and a second end or bottom end 5b opposite to the top end 5a. In general, the receiving part 5 may have a substantially cylindrical outer shape with a central longitudinal axis C extending through the top end 5a and the bottom end 5b. Coaxially to the central axis C, a passage 51 is provided within the receiving part, the passage extending from the top end 5a to the bottom end 5b and forming an opening 52 at the bottom end 5b. At a distance from the top end 5a, the passage 51 widens into an accommodation space 53 that is configured to receive the head 3 and at least a portion of the pressure element 6 therein. Adjacent to the opening 52 at the bottom end 5b, the accommodation space 53 narrows towards the opening 52 in a narrowing portion 53a, which may be, for example, a tapered, and more particularly a conical surface that may cooperate with a corresponding surface of the pressure element 6. The width of the opening 52 may be greater than the greatest diameter of the head 3, so that the head 3 may be inserted from the bottom end 5b into the accommodation space 53. To enable the insertion of the head 3 from the bottom end 5b, the width of the accommodation space 53 is such that the pressure element 6 can expand therein to permit the insertion of the head 3.

The receiving part 5 further comprises a substantially U-shaped recess 54 starting at the top end 5a and extending in a direction of the bottom end 5b. By means of the U-shaped recess 54, two free legs 55 are formed that define a channel that is open towards the first end 5a for receiving the rod 100. The upper part of the receiving part 5 adjacent the top end 5a that comprises the U-shaped recess 54 thus defines a rod receiving portion configured to receive the rod 100, and the lower part of the receiving part 5 adjacent the bottom end 5b that comprises the accommodation space 53 defines a head receiving portion for accommodating the head 3 of the bone anchoring element 1.

On an inner surface of the legs 55, an internal thread 56 is formed that is configured to cooperate with an outer thread of the locking element 8. A circumferential first groove 57a is provided at the inner wall of the legs 55 at a distance from a bottom 54a of the U-shaped recess 54. The first groove 57a provides a stop for restricting an upward movement of the pressure element 6 towards the first end 5a when the pressure element 6 is assembled with the receiving part 5 and when it is in an insertion position. Between the circumferential first groove 57a and the accommodation space 53, a circumferential second groove 57b is provided for engagement with a portion of the pressure element 6 to secure a pre-locking position of the pressure element 6.

At the inner surface of the receiving part 5, a recessed portion 58 is provided that extends from the first groove 57a in the axial direction towards the bottom end 5b of the receiving part 5. A shape of the recessed portion 58 is complementary to a shape of an outer surface portion of the restraining element 7 to provide for a space to at least partially receive the restraining element 7 therein and allow the restraining element to be axially displaced. The recessed portion 58 ends at a lower end at a distance from the second end 5b of the receiving part 5, thus forming a stop 58b for restricting a downward movement of the restraining element 7 towards the second end 5b.

In the present embodiment, two such recessed portions 58 are provided at the inner surface of the receiving part 5, which recessed portions 58 are offset at 180° from one another, each recessed portion 58 being provided on one of the legs 55 at a circumferential position approximately at the center of the leg 55. A position of the recessed portions 58 in the circumferential direction may be at substantially 90° from a longitudinal axis L of the channel defined by the U-shaped recess 54 for receiving the rod 100.

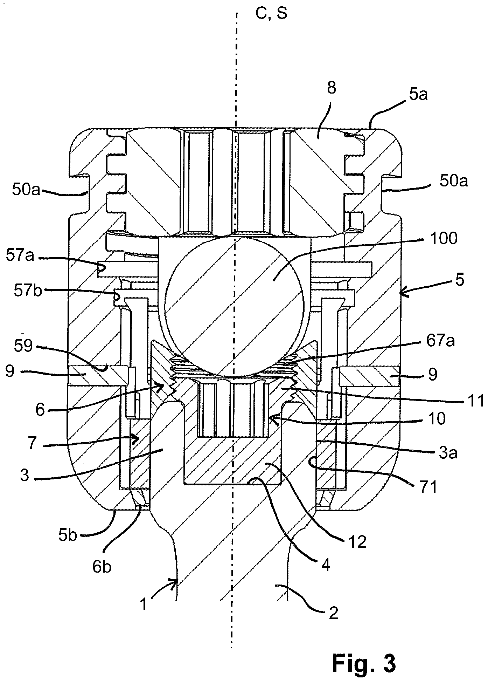

In addition, two through-holes 59 extend through the legs 55 of the receiving part 5 in a direction perpendicular to the central axis C and at a circumferential position approximately at the center of each of the legs 55. An axial position of the through-holes 59 may be approximately at a position of the bottom 54a of the U-shaped recess 54. The through-holes 59 are arranged at 180° offset from each other and at 90° with respect to the longitudinal axis L of the channel defined by the U-shaped recess 54. The through-holes 59 are configured to receive the pins 9 therein, as shown in Figs. 1 and 3. The pins 9 are configured to extend through the through-holes 59 and to engage the restraining elements 7 to secure the restraining elements 7 and the pressure element 6 against rotating.

At the outer surface of each of the legs 55 a circumferential groove 50a may be provided for engagement with a tool or an instrument. The groove 50a may be provided a distance from the top end 5a at the rod receiving portion of the receiving part 5. Alternatively or in addition, notches 50b may be provided at the circumferential sides of the legs facing the U-shaped channel 54, which notches 50b may serve for engagement with a tool or an instrument.

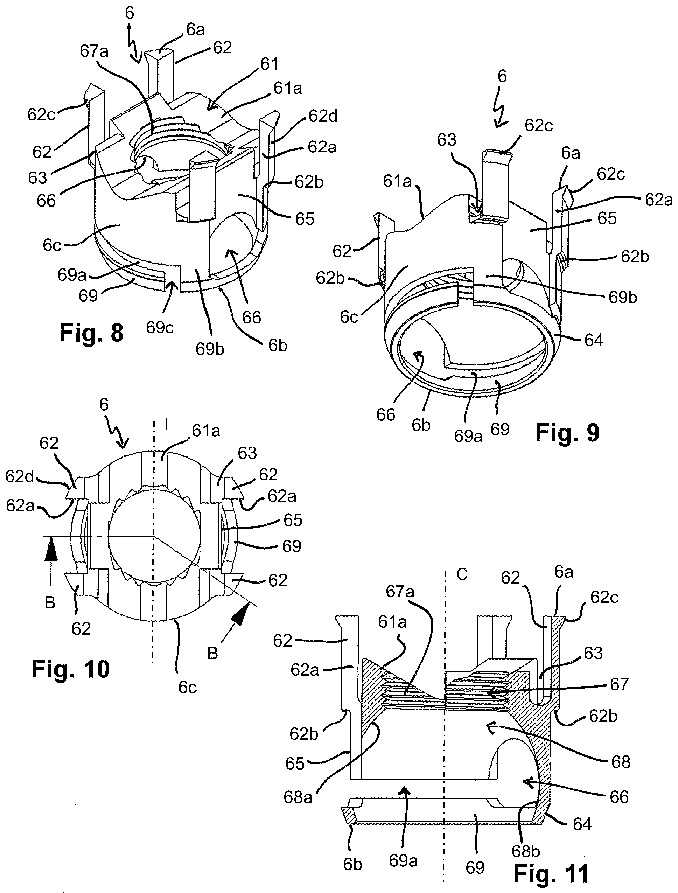

Referring further to Figs. 8 to 11, the pressure element 6 is described. The pressure element 6 may be a monolithic piece that is configured to be arranged in the passage 51 of the receiving part 5 and to exert pressure onto the head when the head 3 and the pressure element 6 are in the receiving part 5 such that the head is pivotable with respect to the receiving part and can be clamped and/or locked at a particular angle relative to the receiving part.

The pressure element 6 has a first end or top end 6a and a second end or bottom end 6b, and may be substantially cylindrical with an outer diameter that allows the pressure element 6 to move in the passage 51 of the receiving part 5. At the top end 6a, a rod receiving recess 61 is formed with a rod support surface 61a. The rod support surface 61a may have a substantially V-shaped cross-section with a longitudinal axis l extending substantially perpendicular to a cylinder axis of the pressure element 6 which coincides with the central axis C of the receiving part 5 when the pressure element 6 is in the receiving part 5. The depth of the rod receiving recess 61 may be smaller than a diameter of the rod 100. Hence, when the rod 100 rests on the rod support surface 61a, the rod 100 projects over the top end 6a of the pressure element as shown, for example, in Fig. 3. The V-shape of the rod support surface 61a facilitates use of rods with different diameters.

The pressure element comprises a central bore extending from the rod receiving recess 61 to the bottom end 6b, the bore comprising an upper portion 67 adjacent the rod receiving recess 61 that widens into a head receiving portion 68 adjacent the bottom end 6b. The upper portion 67 of the bore may be substantially cylindrical with a diameter smaller than a diameter of the head receiving portion 68. Optionally, an engagement structure in the form of an internal thread 67a is provided at the inner surface of the upper portion 67 that is configured to cooperate with an external thread provided at the plug member 10. The head receiving portion 68 is configured to receive the head 3 of the bone anchoring element 1 therein and forms an opening at the bottom end 6b for inserting the head 3. In greater detail, the head receiving portion 68 comprises an upper spherical section 68a adjacent the upper portion 67 of the central bore that widens towards the bottom end 6b and a lower spherical section 68b at the bottom end 6b that narrows towards the bottom end 6b. The upper and lower spherical sections 68a, 68b are shaped so as to matingly receive the spherical head 3 therein and to encompass the head 3 laterally and from a free end of the head and to cover a portion of the head including a largest diameter of the head.

The pressure element 6 further comprises four arms 62 that are arranged in pairs on opposite sides of the rod receiving recess 61. A distance between two of the arms 62 on the same side of the rod receiving recess 61 is such that the restraining element 7 can be received between the two arms 62 and preferably contacts respective sidewalls 62a of the arms 62 to be guided by the sidewalls 62a when displaced in the axial direction. Each arm 62 extends between the top end 6a of the pressure element 6 and a bottom end 62b of the arm 62 along the central axis C with the bottom end 62b being closer to the bottom end 6b of the pressure element 6 than the rod support surface 61a.

Each arm 62 is provided further radially away from the central axis C than a cylindrical outer surface portion 6c of the pressure element 6 so that a gap 63 is formed between the arm 62 and the outer surface portion 6c of the pressure element 6. Hence, by the bottom end 62b of the arm 62, a step may be formed where the arm protrudes away from the outer surface portion 6c of the pressure element 6. The gap 63 formed between the arm 62 and the outer surface portion 6c of the pressure element 6 renders the arm 62 resilient in a direction transverse to the longitudinal axis l of the rod support surface 61a.

A free end of the arms 62 may have a radially protruding rim 62c, an upper surface of which forms the top end 6a of the pressure element 6. The rim 62c is configured to engage the circumferential grooves 57a, 57b provided at the inner surface of the legs 55 of the receiving part 5 to secure the insertion position or the pre-locking position of the pressure element 6 in the receiving part 5. An outer surface 62d of the arms may have a contour of a portion of a cylinder surface.

Adjacent to the bottom end 6b of the pressure element 6, an outer surface portion 64 may be tapered, preferably narrowing in a conical shape, which narrowing outer surface portion 64 is configured to cooperate with the narrowing portion 53a of the receiving part 5.

Between the two arms 62 on the same side of the rod receiving recess 61 and on each side of the rod receiving recess, the outer surface of the pressure element 6 comprises substantially planar or flat surface portions 65. The planar surface portions 65 are recessed from the cylindrical outer surface portion 6c of the pressure element to locally narrow a width of the pressure element 6. The recessed planar surface portions 65 together with the recessed portion 58 at the inner surface of the receiving part 5 provide for a space for receiving the restraining elements 7 therein and permit the restraining element 7 to be axially displaced relative to the pressure element 6.

Moreover, the two planar surface portions 65 are provided on opposite sides of the longitudinal axis l of the pressure element 6 and a plane of each planar surface portion 65 may be substantially parallel to the longitudinal axis l and the central axis C. Each planar surface portion 65 extends from the rod support surface 61a in the axial direction and ends at or above the narrowing outer surface portion 64 at a distance from the bottom end 6b of the pressure element 6.

An opening 66 is provided at the lower end of each planar surface portion 65, the opening 66 providing access to the head receiving portion 68 and to an inserted head from a direction transverse to the central axis C. More specifically, the opening 66 permits a portion of the restraining element 7 to directly contact the head 3 that is received within the head receiving portion 68, as shown in Fig. 3. The opening 66 may have a semi-circular contour with a straight line delimiting the opening 66 towards the bottom end 6b. The contour may be provided with rounded edges of the semi-circle. An axial position of the opening 66 is such that when the head 3 is received within the accommodation space of the receiving part, the planar surface portions 3a of the head 3 are located in a region of the openings 66 of the pressure element 6, as shown in Fig. 3.

At a distance from the second end 6b, a circumferentially extending slit 69a is provided. The slit 69a extends around the central axis C of the pressure element 6 along a plane substantially perpendicular to the central axis C. By means of the slit 69a, a ring-shaped portion 69 of the pressure element 6 is formed at its bottom end 6b. The outer surface of the ring-shaped portion 69 forms the narrowing outer surface 64 of the pressure element 6. The ring-shaped portion 69 may also serve as a stop for limiting a movement of the restraining element 7 towards the bottom end 6b of the pressure element 6.

The slit 69a may extend along the circumference of the pressure element 6 starting from a first one of the openings 66 and extending through the second one of the openings 66 and ending at a distance from the first opening 66 so that a wall portion forming a connection strip 69b is formed between the first opening 66 and the slit 69a. The connection strip 69b forms the only connection portion where the ring-shaped portion 69 is integrally connected to the rest of the pressure element 7. The connecting strip 69b has such a length in the circumferential direction that it provides a stable connection of the ring-shaped portion 69 to the rest of the pressure element 6. At one end of the circumferentially extending slit 69a, there is a substantially vertical slit 69c that extends from the second end 6b fully through the ring-shaped portion 69 into the circumferentially extending slit 69a. The vertical slit 69c together with the ring-shaped portion 69 thus forms a slit ring that is expandable and compressible in the radial direction. When the head 3 of the bone anchoring element 1 is inserted through the opening at the bottom end 6b of the pressure element 6 into the head receiving portion 68, the slit ring expands so that the width of the vertical slit 69c increases, and when the head 3 has been fully inserted into the head receiving portion 68, the slit ring encompasses the head 3 below a position of a largest outer diameter of the head 3 in a direction towards the shank 2.

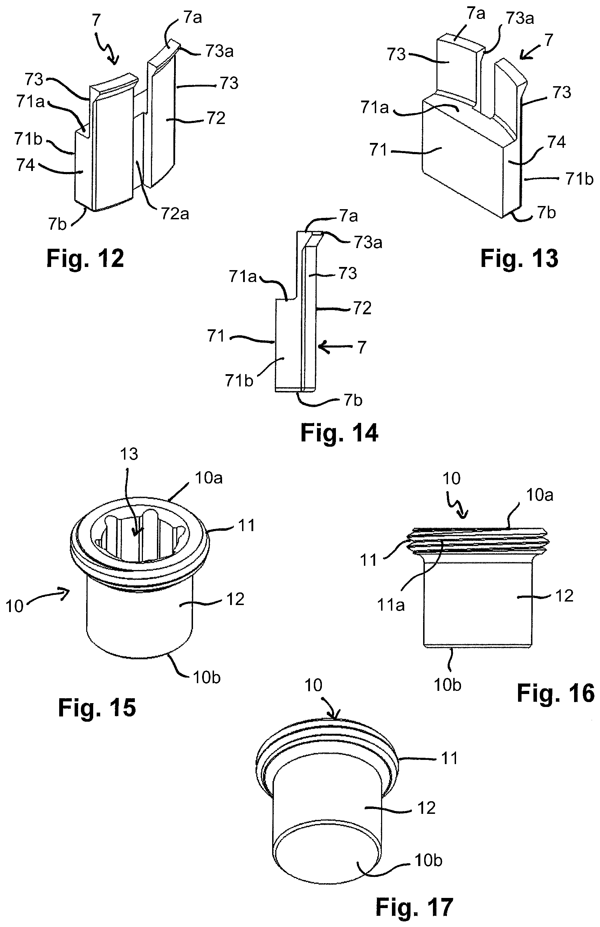

Referring now to Figs. 12 to 14, the restraining element 7 is described. The restraining element 7 preferably is a monolithic part having first end or top end 7a and a second end or bottom end 7b, an outer side facing the receiving part 5 and an opposite inner side facing the pressure element 6 when the restraining element 7 is assembled within the passage 51 of the receiving part 5. The restraining element 7 forms a sliding piece configured to be accommodated in the space formed between the planar surface portion 65 of the pressure element 6 and the recessed portion 58 of the receiving element 7 and to be axially displaced therein.

A length of the restraining element 7 between the top end 7a and the bottom end 7b is such that when the restraining element 7 is in a first or uppermost position with respect to the pressure element 6, wherein the top ends 6a, 7a of the pressure element 6 and the restraining element 7 are at the same axial position, the bottom end 7b of the restraining element 7 is located above the opening 66 of the pressure element 6. When the restraining element 7 is at a second or lowermost position with respect to the pressure element 6, wherein the bottom end 7b of the restraining element abuts on the ring-shaped portion 69 of the pressure element 6 and on the stop 58b formed by the recessed portion 58 of the receiving part 5, the restraining element 7 at least covers the opening 66 of the pressure element 6.

The restraining element 7 generally comprises a base portion 71b extending from the bottom end 7b towards the top end 7a and ending at a distance from the top end 7a, and two arms 73 extending from the base portion 71b to the top end 7a. At the inner side of the restraining element 7, the base portion 71b comprises a first restraining surface in the form of a substantially planar or flat surface portion 71 that is configured to cooperate with the second restraining surface 3a of the head 3. The arms 73 are thinner than the base portion 71b, thus forming a step 71a at the inner side of the restraining element where the arms 73 protrude from the base portion 71b.

The outer side of the restraining element 7 comprises an outer surface portion 72 having a shape of a cylinder surface portion. When the restraining element 7 and the pressure element 6 are assembled in the passage 51 of the receiving part, the outer surface portion 72 of the restraining element 7 is preferably substantially flush with the outer surface 62d of the arms 62 of the pressure element.

A free end of the arms 73 may have a radially protruding rim 73a, an upper surface of which forms the top end 7a of the restraining element 7. The rim 73a may be similar to the rim 62c of the arms 62 of the pressure element 6 and may be configured to engage the circumferential grooves 57a, 57b provided at the inner surface of the legs 55 of the receiving part 5. The outer surface portion 72 of the restraining element 7 further includes a recessed surface portion 72a extending axially from the step 71a to the bottom end 7b in a region between the two arms 73. A shape of the recessed surface portion 72a when seen in a plane of the outer side of the retaining element may be substantially rectangular. The recessed surface portion 72a serves for receiving a front end of the pin 9 therein and to guide the front end of the pin 9 in the recessed surface portion 72a when the restraining element 7 is displaced axially relative to the receiving part 5 and the pressure element 6. The engagement of the recessed surface portion 72a by the pin may prevent rotation of the restraining element 7 and the pressure element 6 relative to the receiving part 5.

Lateral sidewalls 74 of the restraining element 7 that extend between the top end 7a and the bottom end 7b may be substantially flat and configured to contact the respective sidewalls 62a of the arms 62 of the pressure element 6. Specifically, a width of the restraining element 7 between the two sidewalls 74 may be such that the sidewalls 74 contact the respective sidewalls 62a of the arms 62 of the pressure element 6 when the pressure element 6 and the restraining element 7 are assembled within the receiving part 5.

Referring now to Figs. 15 to 17, the plug member 10 is described. The plug member 10 preferably is a monolithic part having first end or top end 10a and a second end or bottom end 10b. At the top end 10a of the plug member 10, a rim portion 11 is formed that has a diameter substantially corresponding to a diameter of the upper portion 67 of the bore of the pressure element 6 and comprises an engagement structure in the form of an outer thread 11a that is configured to cooperate with the inner thread 67a of the pressure element 6. The plug member 10 further comprises a substantially cylindrical portion 12 protruding from the rim portion 11 and terminating in a flat circular face that forms the bottom end 10b of the plug member 10. The cylindrical portion 12 is shaped so as to be received within the engagement recess 4 of the head 3 of the bone anchoring element 1 and has a smaller diameter than the rim portion 11 so that a stop is formed between the rim portion 11 and the cylindrical portion 12 that serves as an abutment for the free end of the head 3, as shown in Fig. 3. The plug member 10 further comprises at its top end 10a an engagement recess 13 for engagement with a tool, such as a driver.

The parts and portions of the bone anchoring device may be made of any material, preferably, however, of titanium or stainless steel or any bio-compatible metal or metal alloy or plastic material. For bio-compatible alloys, a NiTi alloy, for example, Nitinol, may be used. Other materials that can be used are, for example, magnesium or magnesium alloys. Bio-compatible plastic materials that can be used may be, for example, polyether ether ketone (PEEK) or poly-L-lactide acid (PLLA). The parts can be made of the same or of different materials from one another.

Use of the bone anchoring device is described with additional reference to Figs. 18a to 18d. In use, the bone anchoring device can be applied as a polyaxial bone anchoring device or as a uniplanar bone anchoring device or as a monoaxial bone anchoring device.

As shown in Fig. 18a, the pressure element 6 and the two restraining elements 7 are preassembled with the receiving part 5 such that the pressure element 6 is provided in the passage 51 of the receiving part 5 and the restraining elements 7 are accommodated between the recessed portions 58 at the inner surface of the receiving part 5 and the pressure element 6, and the sidewalls 74 of the restraining elements contact the sidewalls 62a of the arms 62 of the pressure element 6. The top ends 6a, 7a of the pressure element 6 and the restraining elements 7 are substantially at the same axial position so that the planar surface portions 71 of the restraining elements 7 contact the planar surface portions 65 of the pressure element 6 and are located axially above the openings 66 of the pressure element. Both the upper rims 62c and 73a of the arms 62, 73 of the pressure element 6 and the restraining elements 7 are held in the first groove 57a at the inner surface of the receiving part, which is the insertion position of the pressure element 6. The pins 9 extend through the through-holes 59 of the receiving part 5 into the recessed surface portions 72a of the restraining elements 7. Thereby, the restraining elements 7 and the pressure element 6 are secured against rotation within the receiving part 5.

With the pressure element 6 held in the insertion position, the head 3 of the bone anchoring element 1 is inserted from the bottom end 5b into the head receiving portion 68 of the pressure element 6. The head 3 is inserted in a position where the substantially planar surface portions 3a are circumferentially aligned with the openings 66 provided at the pressure element 6 and the substantially planar surface portions 71 of the restraining elements 7. This position may be indicated by indication marks provided at the head and the receiving part (not shown in the figures). During insertion, the ring-shaped portion 69 expands to allow the head 3 to enter the head receiving portion 68, and when the head 3 is fully inserted into the head receiving portion 68, the ring-shaped portion 69 encompasses the head 3 below a position of its largest outer diameter, as shown in Fig. 18b. When the head 3 is inserted into the head receiving portion 68 of the pressure element 6, the pressure element 6 and the restraining elements 7 are prevented from moving further towards the top end 5a of the receiving part by the upper rims 62c and 73a of their arms 62, 73 abutting on the upper edge of the first groove 57a.

The pressure element 6 together with the restraining elements 7 is then moved towards the bottom end 5b of the receiving part 5 to assume a pre-locking position where the outer rims 62c, 73a of their arms 62, 73 are held in the second groove 57b of the receiving part 5 and the narrowing outer surface portion 64 at the bottom end 6b of the pressure element 6 engages the narrowing inner surface portion 53a of the receiving part 5 as shown in Fig. 18c. The ends of the pins 9 slide along the recessed surface portions 72a of the restraining elements 72a when the pressure element 6 together with the restraining elements 7 moves axially downward. In the pre-locking position, the head 3 cannot be removed through the lower opening 52 but is still pivotable in multiple different planes relative to the receiving part 5. When the bone anchoring device shall be used as a polyaxial bone anchoring device, the rod 100 is inserted into the U-shaped recess 54 until it rests on the rod support surface 61a of the pressure element 6. To lock the bone anchoring device, i.e. the head 3 and the rod 100 in the receiving part 5, the locking element 8 is screwed between the legs 55 until it presses onto the rod 100 so that the pressure element 6 is moved further downward until it clamps the head 3 at a particular angular position with respect to the receiving part 5.

When the bone anchoring device shall be used as a uniplanar bone anchoring device, the restraining elements 7 are moved from their uppermost or first position shown in Fig. 18c towards the bottom end 6b of the pressure element 6 to assume their lowermost or second position shown in Fig. 18d prior to insertion of the rod 100. For axial displacement of the restraining elements 7 an instrument (not shown in the figures) may be used that is configured to enter to passage 51 of the receiving part and to press on both restraining elements 7 from above to move the restraining elements towards the bottom ends 5b, 6b of the receiving part and the pressure element. During the downward movement, the restraining elements 7 are guided and slide along the planar surfaces 65 and the sidewalls 62a of the arms 62 of the pressure element and the recesses portions 58 at the inner surface of the receiving part until their bottom ends 7b abut against the ring-shaped portion 69 of the pressure element and the stop 58b formed by the recessed portion 58 of the receiving part 5. In this position of the restraining elements 7, the planar surface portions 71 of the restraining elements 7 are at the axial position of the openings 66 of the pressure element 6 so that the planar surface portions 71 directly contact the planar surface portions 3a of the head 3. Pivoting of the head 3 relative to the receiving part 5 is thus restricted to a single plane parallel to the planar surface portions 71, 3a that form the restraining surfaces. In other words, the single plane is spanned by the central axis C and the channel axis L. Again, the rod 100 can be inserted and to lock the head 3 and the rod 100 in the receiving part 5, the locking element 8 is screwed between the legs 55 of the receiving part 5 until it presses onto the rod 100 so that the pressure element 6 is moved further downward until it locks the head 3 at a particular angular position with respect to the receiving part 5 and also locks the rod.

In an alternative manner of use, the restraining elements 7 can be moved to the lowermost position already prior to insertion of the head 3 into the receiving part 5 and the pressure element 6. Thus, the bone anchoring element 1 can be inserted into bone prior to mounting of the receiving part 5 with the pressure element onto the head 3.

When the bone anchoring device shall be used as a monoaxial bone anchoring device, i.e. when the axis of the shank 2 is permanently coaxial with the central axis C of the receiving part 5, the plug member 10 used. Before the rod 100 is inserted, the plug member 10 is assembled with the coupling device by screwing the plug member into the inner thread 67a of the pressure element 6, wherein a tool, such as a driver, may be used. In the inserted position of the plug member 10, as shown in Fig. 3, the cylindrical portion 12 is received within the engagement recess 4 of the head 3 of the bone anchoring element 1 so that the shank 2 of the bone anchoring element 1 is prevented from pivoting relative to the receiving part 5 and assumes a zero-angle position relative to the receiving part 5, i.e. the shank axis S of the shank 2 is coaxial with the central axis C. Again, the rod 100 can be inserted and the head 3 and the rod 100 can be locked in the receiving part 5 by screwing the locking element 8 between the legs 55 of the receiving part 5 and tightened.

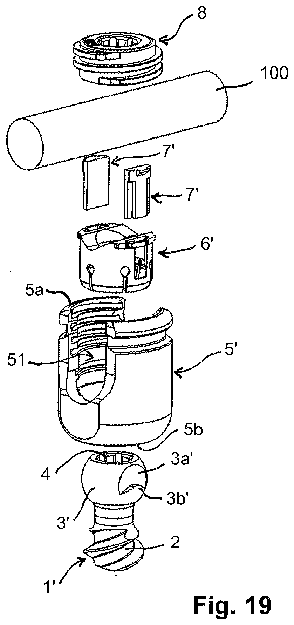

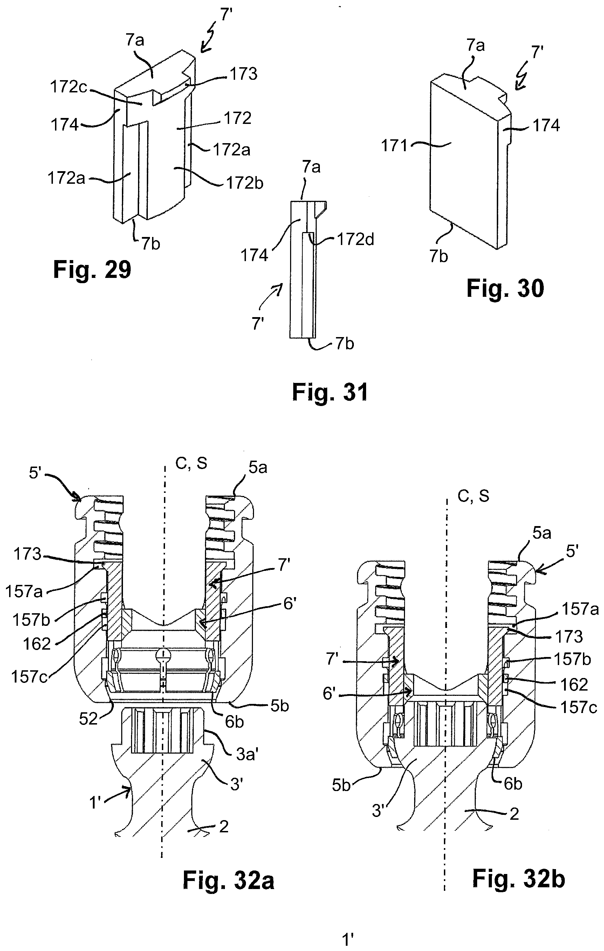

Referring to Figs. 19 to 32d, a second embodiment of the bone anchoring device is described. Parts and portions that are identical or highly similar to the first embodiment are provided with the same reference numerals and the description thereof is not repeated.

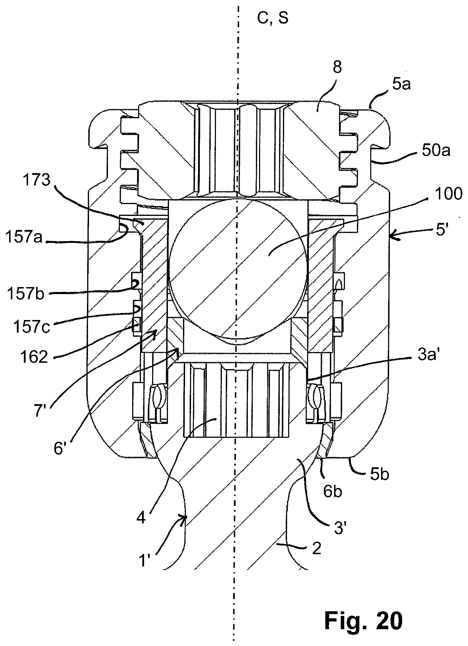

As shown in particular in Figs. 19 and 20, the bone anchoring device of the second embodiment includes a bone anchoring element 1', a receiving part 5', a pressure element 6' and two restraining elements 7', the pressure element 6' and restraining elements 7' being configured to be received within the passage 51 that extends from the top end 5a to the bottom end 5b of the receiving part 5'. As in the first embodiment, the receiving part 5', which may be a monolithic piece, is configured to receive the head 3' of the bone anchoring element 1' and the rod 100 therein, and the pressure element 6' is configured to exert pressure onto the head 3' to clamp the head 3' at a particular angular position relative to the receiving part 5'. The restraining elements 7' are movable relative to the pressure element 6' between a first position and a second position in the axial direction. In the first position, the restraining elements 7' do not act upon the inserted head 3'. Thus, in the first position of the restraining elements 7', the shank 2 of the bone anchoring element 1' may be pivotable in multiple different planes relative to the receiving part 5', i.e., the resulting bone anchoring device is a polyaxial bone anchoring device, and in the second position, the restraining elements 7' may directly contact the inserted head 3' to restrict pivoting of the shank 2 relative to the receiving part 5' within a single plane, i.e., the resulting bone anchoring device is a uniplanar bone anchoring device. To lock the head 3' and the rod 100 in the receiving part 5', a locking element 8 in the form of an inner screw or set screw is provided.

The head 3' of the bone anchoring device of the second embodiment may differ from that of the first embodiment with regard to the shape of the second restraining surfaces. In detail, the second restraining surfaces comprise substantially planar or flat surface portions 3a' provided with a V-shaped or rounded protrusion 3b' at a lower end thereof that may facilitate or assist in pivoting of the head 3' relative to the receiving part 5' in the single plane defined by the first and second restraining surfaces.

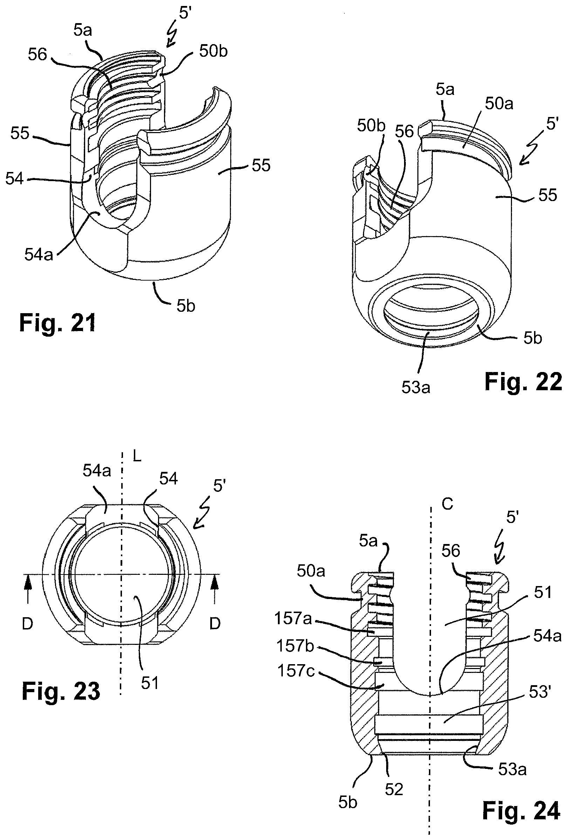

Referring in addition to Figs. 21 to 24, the receiving part 5' comprises the passage 51 that widens into the accommodation space 53' with the narrowing portion 53a provided at the bottom end 5b that narrows towards the opening 52 and cooperates with a corresponding surface of the pressure element 6'. The width of the accommodation space 53' is such that the pressure element 6' can expand therein to permit the insertion of the head 3'.

On the inner surface of the legs 55, a circumferential first groove 157a is provided at a distance from the bottom 54a of the U-shaped recess 54. The first groove 157a is provided for engagement with a portion of the restraining elements 7' to hold the restraining elements 7' in an uppermost or first position with respect to the pressure element 6'. At an axial distance from the first groove 157a and further towards the bottom end 5b of the receiving part 5', a circumferential second groove 157b is provided at the inner surface of the legs that serves for engagement with a portion of the restraining elements 7' to hold the restraining elements 7' in a lowermost or second position with respect to the pressure element 6'. Axially further towards the bottom end 5b and between the circumferential second groove 157b and the accommodation space 53', a circumferential third groove 157c is provided for engagement with a portion of the pressure element 6' to secure an insertion position of the pressure element 6'. The third groove 157c has an axial dimension that permits the engaged portion of the pressure element 6' to move therein between an insertion position and a pre-locking position.

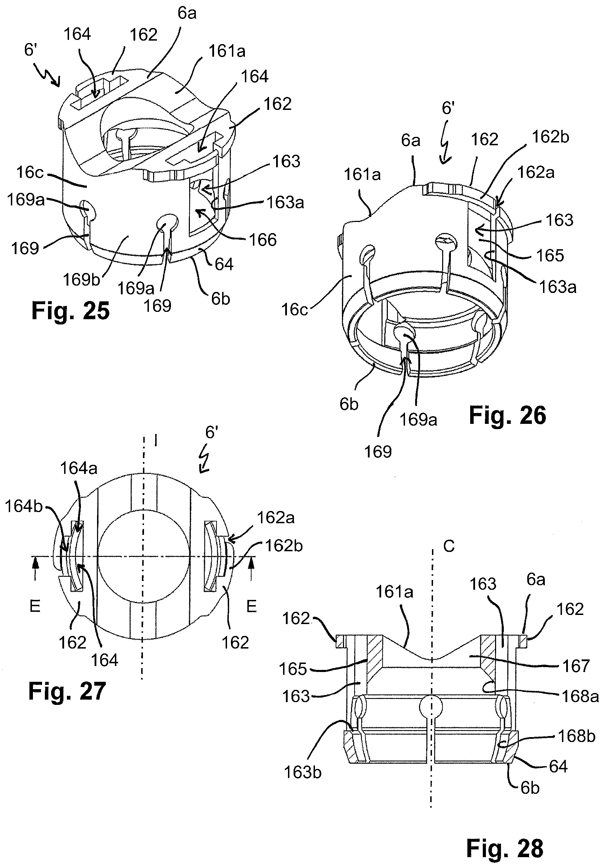

Referring further to Figs. 25 to 28, the pressure element 6' is described. The pressure element 6' preferably is a monolithic piece that is configured to be arranged in the passage 51 of the receiving part 5' and to exert pressure onto the inserted head 3'.

The pressure element 6' has a first end or top end 6a and a second end or bottom end 6b, and may be substantially cylindrical with a cylindrical outer surface portion 16c and an outer diameter that allows the pressure element 6' to move in the passage 51 of the receiving part 5'. In greater detail, the pressure element 6' comprises a rod support surface 161a and two laterally extending protrusions 162 at the top end 6a, a central bore extending from the rod support surface 161a to the bottom end 6b along a cylinder axis of the pressure element 6'. A narrowing outer surface portion 64 is provided adjacent to the bottom end 6b that may be tapered and is configured to cooperate with the narrowing portion 53a of the receiving part 5'. The pressure element 6' further comprises two pockets 163 each of which is configured to receive at least a portion of the restraining element 7' therein.

The rod support surface 161a may have a substantially V-shaped cross-section with a longitudinal axis l extending substantially perpendicular to a cylinder axis of the pressure element 6' which coincides with the central axis C of the receiving part 5' when the pressure element 6' is in the receiving part 5'.

The two protrusions 162 are provided on opposite sides of the longitudinal axis l. Each protrusion extends radially outward from the cylindrical outer surface portion 16c. When the pressure element 6' is assembled within the passage 51 of the receiving part 5', the protrusions are configured to engage the circumferential third groove 157c of the receiving part 5' and are movable within the circumferential third groove 157c in the axial direction. An upper surface of the protrusion 162 forms the top end 6a of the pressure element 6'.

The protrusion 162 comprises an opening 164 that provides access to the pocket 163 for inserting the restraining element 7' into the pocket 163. The opening 164 has a shape that corresponds to a contour of the restraining element 7' when seen in a cross-section of the restraining element 7', the cross-section taken in a plane perpendicular to an insertion direction of the restraining element 7', i.e., perpendicular to the central axis C. In the present embodiment, the opening 164 includes a first portion 164a and a second portion 164b that each have a rectangular contour with the long axis of the rectangle extending parallel to the longitudinal axis l of the rod support surface 161a. The first portion 164a is closer to the rod support surface 161a than the second portion 164b and has a greater dimension parallel to the longitudinal axis l than the second portion 164b so that the opening 164 comprises a stepped portion. The second portion 164b serves to receive a thickened portion of the restraining element 7' therein.

An outer portion of the protrusion 162 that is located farthest away from the longitudinal axis l and is configured to be received within the circumferential third groove 157c of the receiving part 5' may be provided with an axial slit 162a that extends fully through the protrusion 162 in the axial direction and thus forms a spring portion 162b of the protrusion that is resilient in a direction perpendicular to the longitudinal axis l. The edge of the spring portion 162b facing the slit 162a may be rounded.

The central bore of the pressure element 6' comprises an upper portion 167 adjacent the rod support surface 161a that widens into a head receiving portion 168 adjacent the bottom end 6b, wherein the head receiving portion 168 is configured to receive the head 3' of the bone anchoring element 1'. The upper portion 167 may be substantially cylindrical with a diameter smaller than a diameter of the head receiving portion 168. The head receiving portion 168 forms an opening at the bottom end 6b for inserting the head 3' and comprises an upper spherical section 168a and a lower spherical section 168b that are shaped so as to matingly receive the spherical head 3' therein and to encompass the head 3' laterally and from a free end of the head and to cover a portion of the head including a largest diameter of the head.

The head receiving portion 168 comprises a plurality of slits 169 that are open to the bottom end 6b and extend from the bottom end 6b to a distance thereof in the axial direction. Each slit 169 may terminate in an enlarged end portion, such as a circular opening 169a. The slits 169 are spaced apart from one another in a circumferential direction of the pressure element 6', thus forming a plurality of wall sections 169b between them that render the head receiving portion 168 compressible and expandable.

The two pockets 163 of the pressure element 3' are provided on opposite sides of the longitudinal axis l axially below the openings 164 of the protrusions 162. Each pocket 163 extends from the top end 6a of the pressure member in the axial direction and ends at a distance from the bottom end 6b, preferably above the narrowing portion 64. The pocket 163 is accessible through the opening 164 of the protrusion 162 at the top end 6a and is closed towards the bottom end 6b by a stop 163b that may be formed by a wall of the cylindrical outer surface portion 16c of the pressure element 6'.

The pocket 163 is delimited towards the central bore by a substantially planar or flat surface portion 165 that may be similar to the substantially planar or flat surface portion 65 of the pressure element 6 of the first embodiment. Like in the first embodiment, an opening 166 is provided at the lower end of the planar surface portion 165, the opening 166 providing access to the head receiving portion 168 and to an inserted head to permit the first restraining surface at the restraining element 7' to directly contact an inserted head 3'. The opening 166 may have a semi-circular contour with a straight line delimiting the opening 166 towards the bottom end 6b and a circular arc of the contour extending from the straight line towards the top end 6a. The contour may be provided with rounded edges of the semi-circle. An axial position of the opening 166 is such that when the head 3' is received within the accommodation space of the receiving part, the planar surface portions 3a' of the head 3' are located in a region of the openings 166 of the pressure element 6'.

The cylindrical outer surface portion 16c delimits the pocket 163 towards the outside of the pressure element 6'. An opening 163a is provided in the cylindrical outer surface portion 16c that permits a thickened portion of the restraining element 7' to extend there through when the restraining element is received within the pocket 163. The opening 163a has a substantially rectangular contour in a plane of the outer surface of the pressure element and extends from the protrusion 162 to the stop 163b that delimits the pocket to the bottom end 6b. A position and dimension of the opening 163a in the circumferential direction of the pressure element is the same as the position and dimension of the second portion 164b of the opening 164 in the circumferential direction, i.e., parallel to the longitudinal axis l.

Referring now to Figs. 29 to 31, the restraining element 7' is described. The restraining element 7' is a preferably monolithic part having first end or top end 7a and a second end or bottom end 7b, an outer side facing the receiving part 5' and an opposite inner side facing the pressure element 6' when the restraining element 7' is assembled within the passage 51 of the receiving part 5', and sidewalls 174 extending between the inner and outer side and between the top and bottom ends. Generally, the restraining element 7' is a sliding piece configured to be at least partially received within the pocket 163 of the pressure element 6' and to be axially displaced therein.

The restraining element 7' is a substantially rectangular part with a substantially planar or flat surface portion 171 at its inner side that serves as a first restraining surface configured to cooperate with the second restraining surface 3a' of the head 3', and an outer surface portion 172 having a shape of a cylinder surface portion. The outer surface portion 172 comprises two recessed portions 172a each extending from the bottom end 7b towards the top end 7a and ending at a distance from the top end 7a. A shape of the recessed portion 172a when seen in a plane of the outer side of the retaining element may be substantially rectangular. The recessed portions 172a locally reduce a thickness of the restraining element 7' between its inner side and outer side and are located laterally at the outer sides of the outer surface portion 172 adjacent the sidewalls 174, thus forming a first thickened portion 172b between them that is located approximately in the middle of the restraining element 7'. The first thickened portion 172b is configured to be received within the second portion 164b of the opening 164 and the opening 163a of the cylindrical outer surface portion 16c of the pressure element 6', preferably such that the outer surface of the first thickened portion 172b is flush with the cylindrical outer surface portion 16c of the pressure element 6' when the restraining element 7' is received within the pocket 163. The recessed portions 172a of the restraining element 7' are configured to be insertable through the first portion 164a of the opening 164 into the pocket 163 of the pressure element 6'. Generally, a thickness of the restraining element 7' between its inner side and outer side is such that the restraining element 7' is configured to be inserted through the opening 164 and received within the pocket 163 of the pressure element 6'.

Further, by means of the recessed portions 172a ending at a distance from the top end 7a, a second thickened portion 172c is formed adjacent the top end 7a of the restraining element 7', the second thickened portion 172c extending along the entire width of the restraining element 7' between its sidewalls 174. The first and second thickened portions 172b, 172c together form a T-shaped thickened portion of the restraining element 7'. A step 172d is formed between the recessed portions 172a and the second thickened portion 172c, which step 172d is configured to abut on the protrusion 162 of the pressure element 6' when the restraining element 7' is provided in a lowermost or second position with respect to the pressure element 6', e.g., when it is fully inserted into the pocket 163. The step 172d may also serve as a stop for limiting the movement of the restraining element 7' relative to the pressure element 6' towards its bottom end 6b. A dimension of the recessed portions 172a from the bottom end 7b to the step 172d is preferably the same as a dimension of the pocket 163 from the top end 6a of the pressure element 6' to the stop 163b that delimits the pocket towards the bottom end 6b.

In addition, a radially protruding rim or protrusion 173 is provided on the second thickened portion 172c at the top end 7a of the restraining element 7'. The protrusion 173 may be configured to engage the circumferential first and second grooves 157a, 157b provided at the inner surface of the legs 55 of the receiving part 5'. In particular, when the restraining elements 7' are in an uppermost or first position with respect to the pressure element 6', the protrusions 173 may be held in the circumferential first groove 157a of the receiving part 5', and when the restraining elements 7' are in a lowermost or second position with respect to the pressure element 6', the protrusions 173 may be held in the circumferential second groove 157b, to secure the first and second positions, respectively.

Use of the bone anchoring device of the second embodiment is described with additional reference to Figs. 32a to 32d. In use, the bone anchoring device can be applied as a polyaxial bone anchoring device or as a uniplanar bone anchoring device.

As shown in Fig. 32a, the pressure element 6' and the two restraining elements 7' are preassembled with the receiving part 5' such that the pressure element 6' is provided in the passage 51 of the receiving part 5' with its protrusions 162 being held in the third groove 157c at the inner surface of the receiving part 5'. The restraining elements 7' are inserted into the pockets 163 of the pressure element 6' in their uppermost or first positions where their protrusions 173 are held in the first groove 157a at the inner surface of the receiving part and their bottom ends 7b are located axially above the stop 163b of the pocket so that the planar surface portions 171 of the restraining elements 7' are located at the planar surface portions 165 and axially above the openings 166 of the pressure element 6'.

The head 3' of the bone anchoring element 1' is inserted from the bottom end 5b into the head receiving portion 168 of the pressure element 6'. The head receiving portion 168 expands due to the presence of the slits 169 to allow the head 3' to enter the head receiving portion 168. During insertion of the head 3', the pressure element 6' is prevented from moving further towards the top end 5a of the receiving part 5' by the protrusions 162 abutting at the upper edge of the third groove 157c. Fig. 32b shows the state of the bone anchoring device with the head 3' fully inserted.

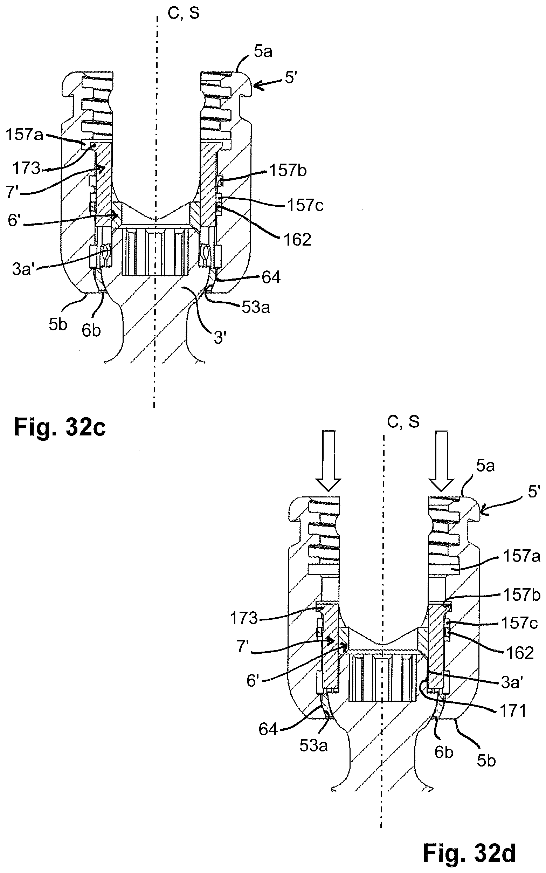

The pressure element 6' is then moved towards the bottom end 5b of the receiving part 5' to assume a pre-locking position where the narrowing outer surface portion 64 at the bottom end 6b of the pressure element 6' engages the narrowing inner surface portion 53a of the receiving part 5' as shown in Fig. 32c. In the pre-locking position, the head 3' cannot be removed through the lower opening 52 but is still pivotable in multiple different planes relative to the receiving part 5'. When the bone anchoring device shall be used as a polyaxial bone anchoring device, the rod 100 is inserted into the U-shaped recess 54 until it rests on the rod support surface 161a of the pressure element 6'. To lock the bone anchoring device, i.e. the head 3' and the rod 100 in the receiving part 5', the locking element 8 is screwed between the legs 55 and tightened to lock the head and the rod.

When the bone anchoring device shall be used as a uniplanar bone anchoring device, the restraining elements 7' are moved from their uppermost or first position shown in Fig. 32c downward into their lowermost or second position shown in Fig. 32d before the rod 100 is inserted. Moving of the restraining elements 7' into the second position can be effected when the pressure element 6' is either in the insertion position or the pre-locking position. For axial displacement of the restraining elements 7' an instrument (not shown in the figures) may be used that is configured to enter to passage 51 of the receiving part and to press on both restraining elements 7' from above to move the restraining elements within the pockets 163 of the pressure element 6' towards the bottom end 6b. The downward movement causes the protrusions 173 to disengage from the first groove 157a of the receiving part 5' and to engage the second groove 157b. During the downward movement, the restraining elements 7' are guided and slide along the planar surfaces 165 within the pockets 163 of the pressure element until their bottom ends 7b abut on the stop 163b at the bottom of the pocket and/or the steps 172d abut on the protrusions 162 of the pressure element 6'. In this position of the restraining elements 7', the planar surface portions 171 are at the axial position of the openings 166 of the pressure element 6' so that the planar surface portions 171 directly contact the planar surface portions 3a' of the head 3'. Pivoting of the head 3' relative to the receiving part 5' is thus restricted to a single plane parallel to the planar surface portions 171, 3a' that form the restraining surfaces. In other words, the single plane is spanned by the central axis C and the channel axis L. Again, the rod 100 can be inserted and the locking element 8 is screwed between the legs 55 of the receiving part 5' and tightened to lock the head and the rod.

Modifications of the above-described embodiments are conceivable. In particular, the shape of the parts is not limited to the detailed shape as shown in the figures. Deviations may be possible and are encompassed by the present disclosure. Although in the embodiments two restraining elements are provided, only one retaining element or more than two retaining elements may be provided. Features described in connection with the bone anchoring device of the first embodiment may also be applied to the second embodiment, and vice versa. For example, the bone anchoring device of the second embodiment may also be provided with the plug member of the first embodiment to obtain a monoaxial bone anchoring device.

Instead of the locking element 8 being a set screw all other kinds of locking assemblies known in the art may be used. For the bone anchoring element all types of bone anchoring elements that are suitable for anchoring in bone or a vertebra, such as bone screws, bone nails, etc. may be used. As used in the present specification and the appended claims, the term "rod" shall be understood as including any elongate member, regardless of the cross-sectional shape of the elongate member. Specifically, a spinal stabilization rod as used herein may have a substantially circular, oval, or angular cross-section. Such a cross-section may further vary along a length of the rod. The rod may be stiff or flexible.

Moreover, the accommodation space of the receiving part and the pressure element may have a design that allows to pivot the bone anchoring element to a greater pivot angle to one side as compared to other sides.

## Claims

1. A bone anchoring device comprising a bone anchoring element (1, 1') having a shank (2) for anchoring to bone and a head (3, 3'), and a coupling device for coupling a rod (100) to the bone anchoring element (1, 1');
wherein the coupling device comprises:
a receiving part (5, 5') having a first end (5a), an opposite second end (5b), and a passage (51) extending between the first end and the second end, the passage (51) defining a central axis (C), wherein the receiving part (5, 5') comprises an accommodation space (53, 53') at the second end (5b) for accommodating the head of the bone anchoring element;
a pressure element (6, 6') configured to be arranged in the passage (51) of the receiving part (5, 5') and to exert pressure onto the head received within the accommodation space (53, 53') to clamp the head at a particular angular position relative to the receiving part,
**characterized in that** the pressure element (6, 6') comprises at least one opening (66, 166) that provides access to the inserted head from a direction transverse to the central axis (C); and
the coupling device further comprises at least one restraining element (7, 7') configured to be arranged in the passage (51) of the receiving part (5, 5'), wherein the at least one restraining element (7, 7') is movable relative to the pressure element (6, 6') in an axial direction between a first position and a second position, wherein in the second position the at least one restraining element contacts the inserted head (3, 3') through the opening (66, 166) of the pressure element (6, 6') to restrict pivoting of the inserted head relative to the receiving part to a single plane.

2. The bone anchoring device of claim 1, wherein when the at least one restraining element (7, 7') is in the first position, the shank (2) of the bone anchoring element (1, 1') is pivotable in multiple different planes relative to the receiving part (5, 5') and/or wherein when the at least one restraining element (7, 7') is in the second position, the at least one restraining element (7, 7') restricts pivoting of the shank of the bone anchoring element relative to the receiving part within a single plane.

3. The bone anchoring device of claim 1 or 2, wherein the at least one restraining element (7, 7') comprises a first restraining surface (71, 171) that is configured to cooperate with a corresponding second restraining surface (3a, 3a') at the head (3, 3') of the bone anchoring element to restrict the pivoting of the bone anchoring element (1, 1').

4. The bone anchoring device of claim 3, wherein when the at least one restraining element (7, 7') is in the first position, the restraining surface (71, 171) of the at least one restraining element (7, 7') does not contact the inserted head, and/or wherein when the at least one restraining element (7, 7') is in the second position, the restraining surface (71, 171) of the at least one restraining element (7, 7') directly contacts the inserted head (3, 3').

5. The bone anchoring device of claim 3 or 4, wherein the first restraining surface comprises a planar surface (71, 171) and/or wherein the head of the bone anchoring element has a substantially spherically-shaped outer surface portion and the at least one second restraining surface comprises a planar surface (3a, 3a') at the outer surface portion of the head.

6. The bone anchoring device of one of claims 1 to 5, wherein the coupling device comprises two restraining elements (7, 7') that are offset from one another by 180°.

7. The bone anchoring device of one of claims 1 to 6, wherein the pressure element (6, 6') and/or the receiving part (5, 5') comprises a guiding structure configured to guide the movement of the at least one restraining element (7, 7') between the first position and the second position, preferably wherein the guiding structure comprises a recessed portion (58) at an inner surface of the receiving part and/or a planar surface (65, 165) at an outer surface of the pressure element and/or a pocket (163) provided at the pressure element.

8. The bone anchoring device of one of claims 1 to 7, wherein the at least one restraining element is entirely provided within the passage of the receiving part, preferably wherein the least one restraining element is received within a space formed between the receiving part and the pressure element and/or in a pocket provided at the pressure element.

9. The bone anchoring device of one of claims 1 to 8, wherein the at least one restraining element (7, 7') is movable from the first position towards the second end (5b) of the receiving part (5, 5') into the second position, and from the second position towards the first end (5a) of the receiving part (5, 5') into the first position,
preferably wherein in the first position, a first end (7a) of the at least one restraining element (7, 7') is at the same axial position as a first end (6a) of the pressure element, and/or preferably wherein a stop (58b, 69, 163b) is provided at the pressure element and/or the receiving part that limits the movement of the at least one restraining element (7, 7') towards the second end (5b) of the receiving part.

10. The bone anchoring device of one of claims 1 to 9, further comprising a plug member (10) that is configured to cooperate with the inserted head of the bone anchoring element such that the plug member (10) forms a monoaxial connection where the bone anchoring element (1, 1') is prevented from pivoting relative to the receiving part (5, 5').

11. The bone anchoring device of claim 10, wherein the pressure element (6) comprises a bore (67) configured to receive a first portion (11) of the plug member (10), the bore preferably comprising an internal thread (67) configured to threadably engage the first portion (11) of the plug member, and/or wherein the plug member (10) comprises a second portion (12), preferably a protrusion, configured to engage a drive recess (4) provided at the head of the bone anchoring element.

12. The bone anchoring device of one of claims 1 to 10, wherein the pressure element (6, 6') comprises a head receiving portion (68, 168) configured to at least partially encompass the head (3, 3') at a circumferential direction thereof, the head receiving portion comprising at least one slit (69a, 69c, 169) that renders the head receiving portion compressible and/or expandable.

13. The bone anchoring device of claim 12, wherein the at least one opening (66, 166) is provided in a wall of the head receiving portion (68, 168) of the pressure element (6, 6').

14. The bone anchoring device of claim 12 or 13, wherein an outer surface (64) of the head receiving portion of the pressure element and an inner surface portion (53a) of the receiving part are configured to cooperate in a manner such that moving the pressure element (6, 6') towards the second end (5b) of the receiving part (5, 5') compresses the head receiving portion (68, 168) of the pressure element to clamp an inserted head in the receiving part, preferably wherein at least one of the cooperating surfaces comprises a tapering shape.

15. The bone anchoring device of one of claims 1 to 14, wherein the pressure element (6, 6') is positionable into a first position in which the head (3, 3') is insertable into the accommodation space (53, 53') and a second position in which the head (3, 3') is prevented from being removed from the accommodation space (53, 53'), preferably wherein the first and/or the second position are secured by a stop (57a, 57b, 157c), and/or
wherein the first and/or the second position of the at least one restraining element is secured by a stop (157a, 157b).

## Patentansprüche

1. Knochenverankerungsvorrichtung, umfassend ein Knochenverankerungselement (1, 1') mit einem Schaft (2) zum Verankern im Knochen und einem Kopf (3, 3'), und eine Kopplungsvorrichtung zum Koppeln eines Stabes (100) an das Knochenverankerungselement (1, 1');
wobei die Kopplungsvorrichtung umfasst:
ein Aufnahmeteil (5, 5') mit einem ersten Ende (5a), einem gegenüberliegenden zweiten Ende (5b) und einem Durchgang (51), der sich zwischen dem ersten Ende und dem zweiten Ende erstreckt, wobei der Durchgang (51) eine Mittelachse (C) definiert, wobei das Aufnahmeteil (5, 5') am zweiten Ende (5b) einen Aufnahmeraum (53, 53') zur Aufnahme des Kopfes des Knochenverankerungselements umfasst;
ein Druckelement (6, 6'), das dazu ausgebildet ist, in dem Durchgang (51) des Aufnahmeteils (5, 5') angeordnet zu sein und Druck auf den im Aufnahmeraum (53, 53') aufgenommenen Kopf auszuüben, um den Kopf in einer bestimmten Winkelposition relativ zum Aufnahmeteil zu halten,
**dadurch gekennzeichnet, dass** das Druckelement (6, 6') zumindest eine Öffnung (66, 166) aufweist, die von einer zur Mittelachse (C) quer verlaufenden Richtung aus Zugang zum eingeführten Kopf bereitstellt; und
die Kopplungsvorrichtung weiter zumindest ein Beschränkungselement (7, 7') umfasst, das dazu ausgebildet ist, im Durchgang (51) des Aufnahmeteils (5, 5') angeordnet zu sein, wobei das zumindest eine Beschränkungselement (7, 7') relativ zum Druckelement (6, 6') in einer axialen Richtung zwischen einer ersten Position und einer zweiten Position bewegbar ist, wobei das zumindest eine Beschränkungselement in der zweiten Position den eingesetzten Kopf (3, 3') durch die Öffnung (66, 166) des Druckelements (6, 6') berührt, um ein Schwenken des eingesetzten Kopfes relativ zum Aufnahmeteil auf eine einzige Ebene zu beschränken.

2. Knochenverankerungsvorrichtung nach Anspruch 1, wobei, wenn das zumindest eine Beschränkungselement (7, 7') in der ersten Position ist, der Schaft (2) des Knochenverankerungselements (1, 1') in mehreren verschiedenen Ebenen relativ zum Aufnahmeteil (5, 5') schwenkbar ist und/oder wobei, wenn das zumindest eine Beschränkungselement (7, 7') in der zweiten Position ist, das zumindest eine Beschränkungselement (7, 7') das Schwenken des Schafts des Knochenverankerungselements relativ zum Aufnahmeteil auf eine einzige Ebene beschränkt.

3. Knochenverankerungsvorrichtung nach Anspruch 1 oder 2, wobei das zumindest eine Beschränkungselement (7, 7') eine erste Beschränkungsfläche (71, 171) umfasst, die dazu ausgebildet ist, mit einer entsprechenden zweiten Beschränkungsfläche (3a, 3a') am Kopf (3, 3') des Knochenverankerungselements zusammenzuwirken, um das Schwenken des Knochenverankerungselements (1, 1') zu beschränken.

4. Knochenverankerungsvorrichtung nach Anspruch 3, wobei, wenn das zumindest eine Beschränkungselement (7, 7') in der ersten Position ist, die Beschränkungsfläche (71, 171) des zumindest einen Beschränkungselements (7, 7') den eingeführten Kopf nicht berührt, und/oder wobei, wenn das zumindest eine Beschränkungselement (7, 7') in der zweiten Position ist, die Beschränkungsfläche (71, 171) des zumindest einen Beschränkungselements (7, 7') direkt mit dem eingeführten Kopf (3, 3') in Kontakt steht.

5. Knochenverankerungsvorrichtung nach Anspruch 3 oder 4, wobei die erste Beschränkungsfläche eine ebene Fläche (71, 171) umfasst und/oder wobei der Kopf des Knochenverankerungselements einen im Wesentlichen sphärisch geformten Außenflächenabschnitt aufweist und die zumindest eine zweite Beschränkungsfläche eine ebene Fläche (3a, 3a') am Außenflächenabschnitt des Kopfes umfasst.

6. Knochenverankerungsvorrichtung nach einem der Ansprüche 1 bis 5, wobei die Kopplungsvorrichtung zwei Beschränkungselemente (7, 7') umfasst, die um 180° zueinander versetzt sind.

7. Knochenverankerungsvorrichtung nach einem der Ansprüche 1 bis 6, wobei das Druckelement (6, 6') und/oder das Aufnahmeteil (5, 5') eine Führungsstruktur umfasst, die dazu ausgebildet ist, die Bewegung des zumindest einen Beschränkungselements (7, 7') zwischen der ersten Position und der zweiten Position zu führen, wobei die Führungsstruktur vorzugsweise einen Ausnehmungsabschnitt (58) an einer Innenfläche des Aufnahmeteils und/oder eine ebene Fläche (65, 165) an einer Außenfläche des Druckelements und/oder eine am Druckelement vorgesehene Tasche (163) umfasst.

8. Knochenverankerungsvorrichtung nach einem der Ansprüche 1 bis 7, wobei das zumindest eine Beschränkungselement vollständig innerhalb des Durchgangs des Aufnahmeteils angeordnet ist, vorzugsweise wobei das zumindest eine Beschränkungselement in einem zwischen dem Aufnahmeteil und dem Druckelement gebildeten Raum und/oder in einer am Druckelement vorgesehenen Tasche aufgenommen ist.

9. Knochenverankerungsvorrichtung nach einem der Ansprüche 1 bis 8, wobei das zumindest eine Beschränkungselement (7, 7') von der ersten Position in Richtung des zweiten Endes (5b) des Aufnahmeteils (5, 5') in die zweite Position bewegbar ist, und von der zweiten Position in Richtung des ersten Endes (5a) des Aufnahmeteils (5, 5') in die erste Position bewegbar ist,
wobei vorzugsweise in der ersten Position ein erstes Ende (7a) des zumindest einen Beschränkungselements (7, 7') an derselben axialen Position wie ein erstes Ende (6a) des Druckelements ist, und/oder wobei vorzugsweise ein Anschlag (58b, 69, 163b) am Druckelement und/oder am Aufnahmeteil vorgesehen ist, der die Bewegung des zumindest einen Beschränkungselements (7, 7') in Richtung des zweiten Endes (5b) des Aufnahmeteils begrenzt.

10. Knochenverankerungsvorrichtung nach einem der Ansprüche 1 bis 9, weiter umfassend ein Stopfenelement (10), das dazu ausgebildet ist, mit dem eingeführten Kopf des Knochenverankerungselements zusammenzuwirken, sodass das Stopfenelement (10) eine monoaxiale Verbindung bildet, bei der ein Schwenken des Knochenverankerungselements (1, 1') relativ zum Aufnahmeteil (5, 5') verhindert ist.

11. Knochenverankerungsvorrichtung nach Anspruch 10, wobei das Druckelement (6) eine Bohrung (67) umfasst, die dazu ausgebildet ist, einen ersten Abschnitt (11) des Stopfenelements (10) aufzunehmen, wobei die Bohrung vorzugsweise ein Innengewinde (67) umfasst, das dazu ausgebildet ist, mit dem ersten Abschnitt (11) des Stopfenelements in Gewindeeingriff zu sein, und/oder wobei das Stopfenelement (10) einen zweiten Abschnitt (12), vorzugsweise einen Vorsprung, umfasst, der dazu ausgebildet ist, mit einer am Kopf des Knochenverankerungselements vorgesehenen Antriebsausnehmung (4) in Eingriff zu sein.

12. Knochenverankerungsvorrichtung nach einem der Ansprüche 1 bis 10, wobei das Druckelement (6, 6') einen Kopfaufnahmeabschnitt (68, 168) umfasst, der dazu ausgebildet ist, den Kopf (3, 3') in dessen Umfangsrichtung zumindest teilweise zu umschließen, wobei der Kopfaufnahmeabschnitt mindestens einen Schlitz (69a, 69c, 169) aufweist, der den Kopfaufnahmeabschnitt zusammendrückbar und/oder ausweitbar macht.

13. Knochenverankerungsvorrichtung nach Anspruch 12, wobei die zumindest eine Öffnung (66, 166) in einer Wand des Kopfaufnahmeabschnitts (68, 168) des Druckelements (6, 6') vorgesehen ist.

14. Knochenverankerungsvorrichtung nach Anspruch 12 oder 13, wobei eine Außenfläche (64) des Kopfaufnahmeabschnitts des Druckelements und ein Innenflächenabschnitt (53a) des Aufnahmeteils dazu ausgebildet sind, derart zusammenzuwirken, dass eine Bewegung des Druckelements (6, 6') in Richtung des zweiten Endes (5b) des Aufnahmeteils (5, 5') den Kopfaufnahmeabschnitt (68, 168) des Druckelements zusammendrückt, um einen in das Aufnahmeteil eingeführten Kopf zu halten, wobei vorzugsweise zumindest eine der zusammenwirkenden Flächen eine sich verjüngende Form umfasst.

15. Knochenverankerungsvorrichtung nach einem der Ansprüche 1 bis 14, wobei das Druckelement (6, 6') in einer ersten Position anordbar ist, in der der Kopf (3, 3') in den Aufnahmeraum (53, 53') einführbar ist, und in einer zweiten Position, in der der Kopf (3, 3') daran gehindert wird, aus dem Aufnahmeraum (53, 53') entfernt zu werden, wobei vorzugsweise die erste und/oder die zweite Position durch einen Anschlag (57a, 57b, 157c) gesichert sind, und/oder
wobei die erste und/oder die zweite Position des zumindest einen Beschränkungselements durch einen Anschlag (157a, 157b) gesichert ist.

## Revendications

1. Dispositif d'ancrage osseux comprenant un élément d'ancrage osseux (1, 1') ayant un manche (2) pour l'ancrage à l'os et une tête (3, 3'), et un dispositif de couplage pour coupler une tige (100) à l'élément d'ancrage osseux (1, 1') ;
dans lequel le dispositif de couplage comprend :
une partie de réception (5, 5') ayant une première extrémité (5a), une seconde extrémité opposée (5b), et un passage (51) s'étendant entre la première extrémité et la seconde extrémité, le passage (51) définissant un axe central (C), dans lequel la partie de réception (5, 5') comprend un espace de logement (53, 53') au niveau de la seconde extrémité (5b) pour loger la tête de l'élément d'ancrage osseux ;
un élément de pression (6, 6') configuré pour être agencé dans le passage (51) de la partie de réception (5, 5') et pour exercer une pression sur la tête reçue à l'intérieur de l'espace de logement (53, 53') pour serrer la tête à une position angulaire particulière par rapport à la partie de réception,
**caractérisé en ce que** l'élément de pression (6, 6') comprend au moins une ouverture (66, 166) qui fournit un accès à la tête insérée à partir d'une direction transversale à l'axe central (C) ; et
le dispositif de couplage comprend en outre au moins un élément de retenue (7, 7') configuré pour être agencé dans le passage (51) de la partie de réception (5, 5'), dans lequel l'au moins un élément de retenue (7, 7') est mobile par rapport à l'élément de pression (6, 6') dans une direction axiale entre une première position et une seconde position, dans lequel dans la seconde position l'au moins un élément de retenue vient en contact avec la tête insérée (3, 3') à travers l'ouverture (66, 166) de l'élément de pression (6, 6') pour limiter le pivotement de la tête insérée par rapport à la partie de réception dans un plan unique.

2. Dispositif d'ancrage osseux selon la revendication 1, dans lequel lorsque l'au moins un élément de retenue (7, 7') est dans la première position, le manche (2) de l'élément d'ancrage osseux (1, 1') peut pivoter dans de multiples plans différents par rapport à la partie de réception (5, 5') et/ou dans lequel lorsque l'au moins un élément de retenue (7, 7') est dans la seconde position, l'au moins un élément de retenue (7, 7') limite le pivotement du manche de l'élément d'ancrage osseux par rapport à la partie de réception dans un plan unique.

3. Dispositif d'ancrage osseux selon la revendication 1 ou 2, dans lequel l'au moins un élément de retenue (7, 7') comprend une première surface de retenue (71, 171) qui est configurée pour coopérer avec une seconde surface de retenue correspondante (3a, 3a') au niveau de la tête (3, 3') de l'élément d'ancrage osseux pour limiter le pivotement de l'élément d'ancrage osseux (1, 1').

4. Dispositif d'ancrage osseux selon la revendication 3, dans lequel lorsque l'au moins un élément de retenue (7, 7') est dans la première position, la surface de retenue (71, 171) de l'au moins un élément de retenue (7, 7') ne vient pas en contact avec la tête insérée, et/ou dans lequel lorsque l'au moins un élément de retenue (7, 7') est dans la seconde position, la surface de retenue (71, 171) de l'au moins un élément de retenue (7, 7') vient directement en contact avec la tête insérée (3, 3').

5. Dispositif d'ancrage osseux selon la revendication 3 ou 4, dans lequel la première surface de retenue comprend une surface plane (71, 171) et/ou dans lequel la tête de l'élément d'ancrage osseux a une partie de surface externe de forme sensiblement sphérique et l'au moins une seconde surface de retenue comprend une surface plane (3a, 3a') au niveau de la partie de surface externe de la tête.

6. Dispositif d'ancrage osseux selon l'une des revendications 1 à 5, dans lequel le dispositif de couplage comprend deux éléments de retenue (7, 7') qui sont décalés l'un de l'autre de 180°.

7. Dispositif d'ancrage osseux selon l'une des revendications 1 à 6, dans lequel l'élément de pression (6, 6') et/ou la partie de réception (5, 5') comprend une structure de guidage configurée pour guider le mouvement de l'au moins un élément de retenue (7, 7') entre la première position et la seconde position, de préférence dans lequel la structure de guidage comprend une partie évidée (58) au niveau d'une surface interne de la partie de réception et/ou une surface plane (65, 165) au niveau d'une surface externe de l'élément de pression et/ou une poche (163) prévue au niveau de l'élément de pression.

8. Dispositif d'ancrage osseux selon l'une des revendications 1 à 7, dans lequel l'au moins un élément de retenue est entièrement prévu à l'intérieur du passage de la partie de réception, de préférence dans lequel l'au moins un élément de retenue est reçu à l'intérieur d'un espace formé entre la partie de réception et l'élément de pression et/ou dans une poche prévue au niveau de l'élément de pression.

9. Dispositif d'ancrage osseux selon l'une des revendications 1 à 8, dans lequel l'au moins un élément de retenue (7, 7') est mobile de la première position vers la seconde extrémité (5b) de la partie de réception (5, 5') dans la seconde position, et de la seconde position vers la première extrémité (5a) de la partie de réception (5, 5') dans la première position,
de préférence dans lequel dans la première position, une première extrémité (7a) de l'au moins un élément de retenue (7, 7') est à la même position axiale qu'une première extrémité (6a) de l'élément de pression, et/ou de préférence dans lequel une butée (58b, 69, 163b) est prévue au niveau de l'élément de pression et/ou de la partie de réception qui limite le mouvement de l'au moins un élément de retenue (7, 7') vers la seconde extrémité (5b) de la partie de réception.

10. Dispositif d'ancrage osseux selon l'une des revendications 1 à 9, comprenant en outre un élément de bouchon (10) qui est configuré pour coopérer avec la tête insérée de l'élément d'ancrage osseux de sorte que l'élément de bouchon (10) forme une connexion monoaxiale où l'élément d'ancrage osseux (1, 1') est empêché de pivoter par rapport à la partie de réception (5, 5').

11. Dispositif d'ancrage osseux selon la revendication 10, dans lequel l'élément de pression (6) comprend un alésage (67) configuré pour recevoir une première partie (11) de l'élément de bouchon (10), l'alésage comprenant de préférence un filetage interne (67) configuré pour mettre en prise par filetage la première partie (11) de l'élément de bouchon, et/ou dans lequel l'élément de bouchon (10) comprend une seconde partie (12), de préférence une saillie, configurée pour mettre en prise un évidement d'entraînement (4) prévu au niveau de la tête de l'élément d'ancrage osseux.

12. Dispositif d'ancrage osseux selon l'une des revendications 1 à 10, dans lequel l'élément de pression (6, 6') comprend une partie de réception de tête (68, 168) configurée pour entourer au moins partiellement la tête (3, 3') au niveau d'une direction circonférentielle de celle-ci, la partie de réception de tête comprenant au moins une fente (69a, 69c, 169) qui rend la partie de réception de tête compressible et/ou expansible.

13. Dispositif d'ancrage osseux selon la revendication 12, dans lequel l'au moins une ouverture (66, 166) est prévue dans une paroi de la partie de réception de tête (68, 168) de l'élément de pression (6, 6').

14. Dispositif d'ancrage osseux selon la revendication 12 ou 13, dans lequel une surface externe (64) de la partie de réception de tête de l'élément de pression et une partie de surface interne (53a) de la partie de réception sont configurées pour coopérer d'une manière telle que le déplacement de l'élément de pression (6, 6') vers la seconde extrémité (5b) de la partie de réception (5, 5') comprime la partie de réception de tête (68, 168) de l'élément de pression pour serrer une tête insérée dans la partie de réception, de préférence dans lequel au moins l'une des surfaces de coopération comprend une forme effilée.

15. Dispositif d'ancrage osseux selon l'une des revendications 1 à 14, dans lequel l'élément de pression (6, 6') peut être positionné dans une première position dans laquelle la tête (3, 3') peut être insérée dans l'espace de logement (53, 53') et une seconde position dans laquelle la tête (3, 3') est empêchée d'être retirée de l'espace de logement (53, 53'), de préférence dans lequel la première et/ou la seconde position sont fixées par une butée (57a, 57b, 157c), et/ou
dans lequel la première et/ou la seconde position de l'au moins un élément de retenue sont fixées par une butée (157a, 157b).
